# EUROPÄISCHE PATENTSCHRIFT

(11) **EP 1 200 251 B1**
(45) Veröffentlichungstag und Bekanntmachung des Hinweises auf die Patenterteilung: **03.09.2003**
(21) Anmeldenummer: 00947867.8
(22) Anmeldetag: 23.06.2000
(51) Int. Cl.: B32B 7/02, A61F 13/15, A61F 5/48, A41D 31/02

(54) **FLÄCHIGER BIEGSAMER ABSORBIERENDER SCHICHT-VERBUNDSTOFF**
FLAT, FLEXIBLE, BONDED COMPOSITE MATERIAL
MATERIAU COMPOSITE STRATIFIE ABSORBANT, PLAN, FLEXIBLE

(30) Priorität: 31.07.1999 DE 19936154
(43) Veröffentlichungstag der Anmeldung: 02.05.2002
(73) Patentinhaber: Deotexis Inc., New York, N.Y. 10022-4838 (US)
(72) Erfinder: TEBBE, Gerold, MC-98000 Monte Carlo (MC)
(74) Vertreter: Ostertag, Reinhard
(86) Internationale Anmeldenummer: EP0005789
(87) Internationale Veröffentlichungsnummer: WO01008880

(56) Entgegenhaltungen:
- WO-A-99/49825
- DE-A- 3 640 374
- US-A- 4 424 247
- US-A- 5 330 817
- US-A- 5 879 487
- DATABASE WPI Section Ch, Week 198431 Derwent Publications Ltd., London, GB; Class A96, AN 1984-191061 XP002151821 & JP 59 106501 A (MITSUI TOATSU CHEM INC), 20. Juni 1984 (1984-06-20)
- DATABASE WPI Section Ch, Week 199422 Derwent Publications Ltd., London, GB; Class D22, AN 1994-176794 XP002151822 & BR 9 204 008 A (MARQUES H M), 12. April 1994 (1994-04-12)

## Beschreibung

Die Erfindung betrifft einen flächigen biegsamen Schicht-Verbundstoff.

Derartige Schicht-Verbundstoffe sind in vielfältiger Ausführung, z.B. als non-woven-fabrics, Filze, Vliesstoffe, Fadenverbundstoffe oder Schichtstoffe (bondings) bekannt.

Bekannte derartige Schicht-Verbundstoffe sind entweder nicht wasserdicht oder haben, wenn sie eine wasserundurchlässige Schicht aufweisen, einen geringen Tragekomfort, da sich auf der dem Benutzer zugewandten Seite des Schicht-Verbundstoffs das Wasser derart sammelt, daß es vom Benutzer als unangenehm empfunden wird.

Zwar sind Textilien mit einem Schicht-Verbundstoff schon bekannt (z.B. Windeln oder Slipeinlagen), diese Textilien haben jedoch nur einen begrenzten Anwendungsbereich. Ein textiler Grundstoff, aus dem nachträglich Textilen für individuelle Anwendungsbereiche hergestellt werden können, ist durch diese bekannten Textilien nicht gegeben.

Es ist daher die Aufgabe der vorliegenden Erfindung, einen Schicht-Verbundstoff der eingangs genannten Art derart weiterzubilden, daß er wasserdicht ist und gleichzeitig einen guten Tragekomfort aufweist.

Diese Aufgabe ist erfindungsgemäß gelöst durch einen Schicht-Verbundstoff mit den im Anspruch 1 angegebenen Merkmalen.

Die Wirkungsweise des erfindungsgemäßen Schichtaufbaus ist derjenigen der oben genannten bekannten Textilien jedoch vergleichbar: Vom Benutzer abgegebene Körperflüssigkeit verteilt sich durch Diffusion in der flüssigkeitsaufnehmenden Schicht, so daß es zu keiner unerwünschten Stauung der Körperflüssigkeit an Kontaktstellen kommt, an denen der Benutzer mit dem Schicht-Verbundstoff in Berührung steht. Die flüssigkeitsundurchlässige Schicht wiederum verhindert ein Durchsickern der Körperflüssigkeit durch den Schicht-Verbundstoff und damit ein Benetzen bzw. Durchnässen anderer Stoffe oder Objekte, die an der vom Benutzer abgewandten Seite des Schicht-Verbundstoffs angeordnet sind.

Die Trägerschicht gewährleistet eine mechanische Verstärkung für die flüssigkeitsundurchlässige Schicht. Sie ist kostengünstig und leicht, ist gleichzeitig aber für Anwendungen, bei denen die Trägerschicht im wesentlichen als Abstandshalter zwischen flüssigkeitsundurchlässiger Schicht und Unterlage des Schicht-Verbundstoffs dient, voll ausreichend.

Durch das Binden der Körperflüssigkeit in der flüssigkeitsaufnehmenden Schicht wird gegebenenfalls auch eine Geruchsbelästigung durch austretende Flüssigkeit vermieden.

Der Schicht-Verbundstoff ist flexibel einsetzbar, z.B. als Betteinlage oder -laken oder als eine ggf. über einer herkömmlichen Windel zu tragende Textilie.

Die Weiterbildung gemäß Anspruch 2 ist eine besonders einfache und kostengünstige Ausführung der flüssigkeitsundurchlässigen Schicht. Je nach verwendetem Folienmaterial (Kunststoff, Metall usw.) kann durch die Folie im Schicht-Verbundstoff auch eine tragende Funktion erfüllt werden. Bei Verwendung dünner Polymer-Folien, z.B. aus PE, lassen sich dünne und schmiegsame Schicht-Verbundstoffe realisieren.

Durch die Ausgestaltung des Schicht-Verbundstoffs gemäß Anspruch 3 wird ein guter Tragekomfort bei hoher Flüssigkeitsaufnahmefähigkeit des Schicht-Verbundstoffs erzielt.

Die abstehenden Fasern des Flors halten dabei die Körperoberfläche des Benutzers auf einen vorgegebenen Abstand zur flüssigkeitsundurchlässigen Schicht, so daß ein Kontakt zwischen Körperoberfläche und flüssigkeitsundurchlässiger Schicht mit der Gefahr eines Flüssigkeitsstaus sicher vermieden ist.

Nochmals erhöht wird sowohl Tragekomfort als auch Flüssigkeitsaufnahmefähigkeit durch die Ausgestaltung gemäß Anspruch 4. Unter einem Flausch wird dabei ein wolliger Flor verstanden, der wesentlich höher ist als der Flor eines normalen Velours-Stoffes.

Ist die flüssigkeitsaufnehmende Schicht gemäß Anspruch 5 ausgestaltet, resultiert ebenfalls neben einem guten Tragekomfort eine hohe Flüssigkeitsaufnahmekapazität.

Bei einer Ausgestaltung des Schicht-Verbundstoffs gemäß Anspruch 6 hat dieser zusätzlich die Funktion der Abgabe eines Wirkstoffs. Die Abgabemenge sowie die Abgabedauer lassen sich dabei über die Stabilität der Hülle der Mikrokapseln steuern. Als Wirkstoffe kommen z.B. die Atmung erleichternde Menthol-Extrakte, Duftstoffe oder geruchskompensierende Stoffe in Frage.

Die Ausgestaltung gemäß Anspruch 7 sorgt dabei für das Aufrechterhalten einer einmal eingestellten gleichmäßigen Verteilung der Mikrokapseln am bzw. im Schicht-Verbundstoff.

Bei der Ausführung gemäß Anspruch 8 ist zusätzlich eine Rutschfestigkeit des Schicht-Verbundstoffs auf einer Unterlage gegeben.

Eine zusätzliche Abdeckschicht gemäß Anspruch 9 führt einerseits zu einem Schutz der flüssigkeitsaufnehmenden Schicht, z.B. gegen mechanische Einwirkung, andererseits besonders dann, wenn die flüssigkeitsaufnehmende Schicht hydrophob ausgeführt ist, dazu, daß der Körper des Benutzers nochmals zusätzlich gegen Flüssigkeitsstauungen in der flüssigkeitsaufnehmenden Schicht isoliert ist.

Die Verbindung der Abdeckschicht mit der flüssigkeitsaufnehmenden Schicht gemäß Anspruch 10 gestattet die Herstellung von Endlos-Schicht-Verbundstoffen mit Abdeckschicht.

Nachstehend wird die Erfindung anhand von Ausführungsbeispielen unter Bezugnahme auf die Zeichnung näher erläutert. In dieser zeigen:
- Figur 1: einen Schnitt durch einen Ausschnitt eines Schicht-Verbundstoffs;
- Figur 2: einen vergrößerten Ausschnitt aus einer Florschicht des Schicht-Verbundstoffs von Fig. 1;
- Figur 3: eine Aufsicht auf einen alternativen Schicht-Verbundstoff, von der Auflageseite her gesehen; und
- Figur 4: eine Aufsicht auf einen nochmals alternativen Schicht-Verbundstoff, von der dem Benutzer zugewandten Seite her gesehen.

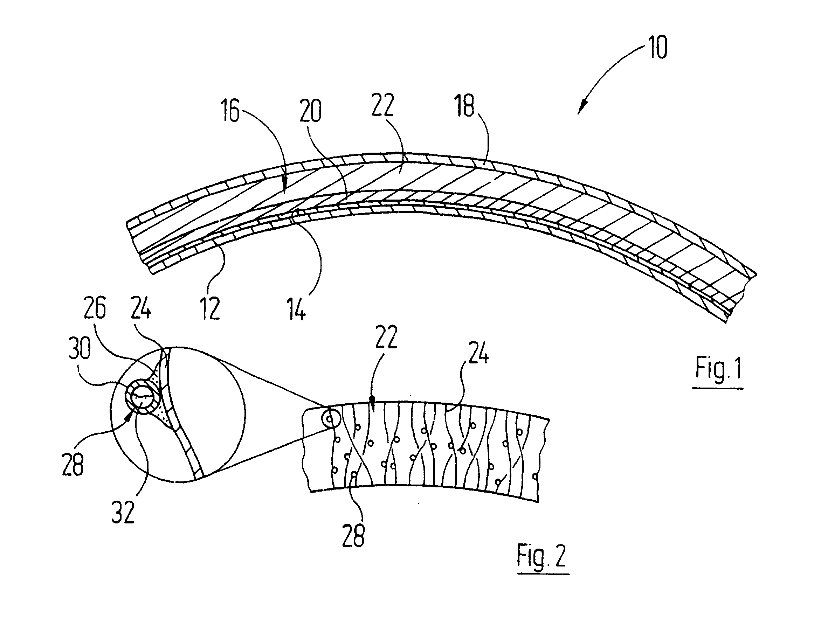

Der in Figur 1 insgesamt mit dem Bezugszeichen 10 bezeichnete Schicht-Verbundstoff ist ein biegsames Flächengebilde, das sich an die Körperkontur eines Benutzers anpassen kann.

Ausgehend von einer Trägerschicht 12, die in der Figur zuunterst dargestellt ist und aus einem widerstandsfähigen hydrophoben Kunstfaser-Material besteht, weist der Schicht-Verbundstoff 10 folgende weitere Schichten auf: Eine flüssigkeitsundurchlässige Folie 14 aus einem Polymermaterial (z.B. PE), eine Flauschschicht 16 aus Baumwolle sowie eine Abdeck-Gewebeschicht 18 aus luft- und flüssigkeitsdurchlässigem Textilmaterial. Die Flauschschicht 16 ist ihrerseits aus zwei Schichten aufgebaut, nämlich aus einer Flausch-Grundschicht 20, mit der die Folie 14 verklebt ist, und einer Florschicht 22.

Die Trägerschicht 12 sowie die Flauschschicht 16 sind flächig mit der Folie 14 verklebt.

Die Florschicht 22 weist in unbelastetem Zustand des Schicht-Verbundstoffs 10 eine Dicke auf, die etwa dreimal so groß ist, wie die der Flausch-Grundschicht 20. Sie ist aus einer Vielzahl einzelner Baumwollfasern 24 (vgl. Fig. 2) aufgebaut, die von der Flausch-Grundschicht 20 abstehen.

Die Abdeck-Gewebeschicht 18 ist mit der Flauschschicht 16 lose verbunden.

Der Aufbau der Flauschschicht 16 wird anhand des in Fig. 2 dargestellten Ausschnitts des Schicht-Verbundstoffs 10 der Fig. 1 deutlich: eine Vielzahl von Baumwollfasern 24, die sich zwischen der Flausch-Grundschicht 20 und der Abdeckschicht 18 erstrecken, bilden die Florschicht 22.

Wie insbesondere die Ausschnittsvergrößerung der Figur 2 im Bereich einer Baumwollfaser 24 verdeutlicht, haften mittels eines Bindemittels 26 Mikrokapseln 28 an den Baumwollfasern 24.

Die Mikrokapseln 28 weisen eine Hülle 30, z.B aus Gelatine auf, in der ein flüssiger Wirkstoff 32 aufgenommen ist. Die Durchlässigkeit der Hülle 30, die durch Druck, Temperatur oder Feuchtigkeit abbau- oder zerstörbr ist, für den Wirkstoff 32 ist nur sehr gering, so daß pro Zeiteinheit nur eine kleine Wirkstoffmenge vom Innenraum der Hülle 30 nach außen dringt.

Alternativ zu einer Flauschschicht 16 kann der Schicht-Verbundstoff 10 auch ein Vlies, z.B. ein Woll-Vlies aufweisen.

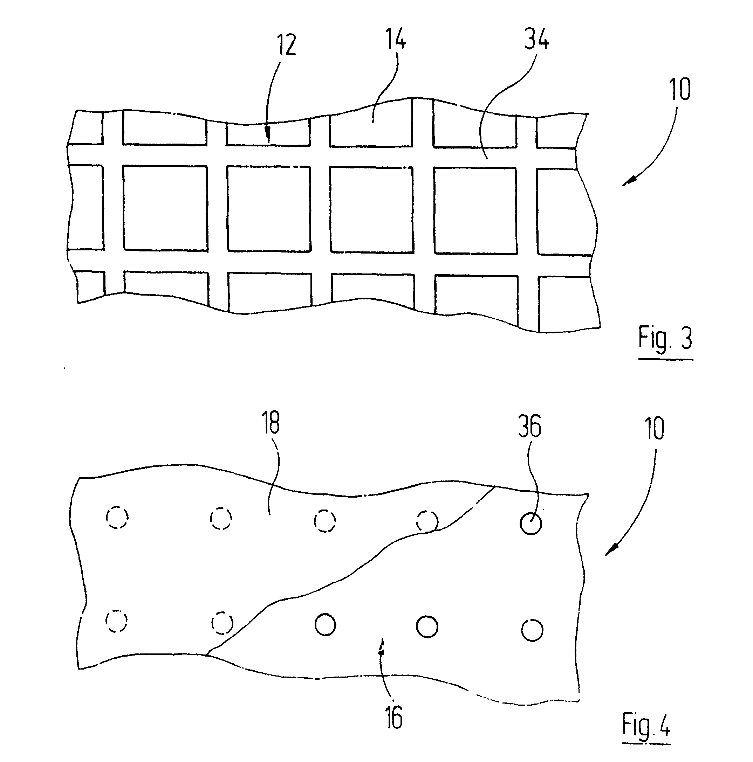

Die Trägerschicht 12 kann bei der Verwendung des Schichtverbundstoffs 10 als Betteinlage auch ein flexibles Netz 34 (vgl. Fig. 3) aus einem Material mit hohem Reibungswert, z.B. Gummi sein, wodurch das Verrutschen des Schicht-Verbundstoffs 10 auf einer Unterlage verhindert wird. Das flexible Netz 34 ist mit der Folie 14 verklebt, kann alternativ aber auch mit dieser verschweißt oder vernäht sein.

Bei der Herstellung von Endlos-Bahnen des Schicht-Verbundstoffs 10 ist die Abdeckschicht 18 nicht als lose Schicht ausgeführt, sondern, wie in Figur 4 dargestellt mit der Flauschschicht 16 verklebt. Zur besseren Anschaulichkeit ist in Figur 4 ein Teil der Abdeckschicht 18 weggelassen, um einen Blick auf die darunterliegende Flauschschicht 16 freizugeben. Die Flauschschicht 16 trägt Klebstoffportionen an einer Mehrzahl von rasterartig angeordneten Klebepositionen 36. Der so angeordnete Klebstoff verklebt die Abdeckschicht 18 punktweise mit der Flauschschicht 16. Auf diese Weise verbleibt auch bei der Verwendung eines undurchlässigen Klebstoffs zwischen den Klebepositionen 36 genügend Fläche, durch die ein Luft- bzw. Feuchtigkeitsaustausch möglich ist.

Alternativ zum Verkleben kann die Folie 14 auch mit der Trägerschicht 12 bzw. der Flauschschicht 16 bzw. dem Vlies verschweißt sein.

Die Funktion des Schicht-Verbundstoffs 10 ist folgendermaßen:

Bei der Benutzung des Schicht-Verbundstoffs 10, z.B. als Betteinlage, Bettlaken oder als Unterwäsche, ist die Abdeck-Gewebeschicht 18 dem Körper des Benutzers zugewandt. Vom Benutzer abgegebene Körperflüssigkeit durchdringt die Abdeck-Gewebeschicht 18 und wird von der Flauschschicht 16 aufgenommen. Dabei verteilt sich die Körperflüssigkeit durch Diffusion großflächig über die Fläche des Schicht-Verbundstoffs 10, so daß im Bereich der Abdeck-Gewebeschicht 18 kein Flüssigkeitsstau, der als unangenehm empfunden wird, entsteht.

Die Folie 14 dient als Flüssigkeitssperre, so daß die Körperflüssigkeit des Benutzers den Schicht-Verbundstoff nicht durchdringen kann.

Durch die Aufnahme der Körperflüssigkeit in der Flauschschicht 16 bzw. in dem Vlies wird die Körperflüssigkeit vom Benutzer abgeführt, so daß dieser trocken liegt.

Eine weitere Anwendung des Schicht-Verbundstoffs 10 liegt in der Langzeitanwendung von flüchtigen Wirkstoffen oder Düften.

Ein durch die Hüllen 30 der Mikrokapseln 28 entweichender flüchtiger Wirkstoff 32, z.B. ein Menthol- oder Kräuterextrakt zur Verbesserung der Durchlässigkeit der Atemwege oder ein Duftstoff, durchdringt über einen langen Zeitraum, der über die Wirkstoffkonzentration und die Durchlässigkeit der Hülle 30 vorgegeben werden kann, die Abdeck-Gewebeschicht 18 und wird vom Benutzer über die Atmung aufgenommen.

## Patentansprüche

1. Flächiger biegsamer Schicht-Verbundstoff mit einer flüssigkeitsundurchlässigen Schicht (14) und einer mit dieser verbundenen flüssigkeitsaufnehmenden Schicht (16), **dadurch gekennzeichnet, daß** eine Trägerschicht (12), bei der es sich um eine flexible Netzschicht (34) handelt, die flüssigkeitsundurchlässige Schicht (14) trägt.

2. Schicht-Verbundstoff nach Anspruch 1, **dadurch gekennzeichnet, daß** die flüssigkeitsundurchlässige Schicht eine Folie (14) ist.

3. Schicht-Verbundstoff nach Anspruch 1 oder 2, **dadurch gekennzeichnet, daß** die flüssigkeitsaufnehmende Schicht (16) einen Flor (22) aufweist, vorzugsweise aus Baumwollfasern (24).

4. Schicht-Verbundstoff nach Anspruch 3, **dadurch gekennzeichnet, daß** die flüssigkeitsaufnehmende Schicht (16) einen Flausch aufweist.

5. Schicht-Verbundstoff nach Anspruch 1 oder 2, **dadurch gekennzeichnet, daß** die flüssigkeitsaufnehmende Schicht (16) eine Vliesschicht aufweist.

6. Schicht-Verbundstoff nach einem der vorhergehenden Ansprüche, **dadurch gekennzeichnet, daß** die flüssigkeitsaufnehmende Schicht (16) Mikrokapseln (28) aufweist, die mit einem Wirkstoff (32) gefüllt sind und eine durch Druck, Temperatur oder Feuchtigkeit abbau- oder zerstörbare Hülle (30) aufweisen.

7. Schicht-Verbundstoff nach Anspruch 6 in Verbindung mit Anspruch 3, **dadurch gekennzeichnet, daß** die Mikrokapseln (28) an den Fasern (24) des Flors (22) haften, vorzugsweise über ein Bindemittel (26).

8. Schicht-Verbundstoff nach einem der Ansprüche 1 bis bis 7, **dadurch gekennzeichnet, daß** die flexible Netzschicht (34) aus einem Material mit hoher Reibung, insbesondere aus Gummi ist.

9. Schicht-Verbundstoff nach einem der vorhergehenden Ansprüche, **dadurch gekennzeichnet, daß** auf der Seite, die von der flüssigkeitsundurchlässigen Schicht (14) abgewandt ist, eine flüssigkeitsdurchlässige, vorzugsweise hydrophobe Abdeckschicht (18) für die flüssigkeitsaufnehmende Schicht (16) vorgesehen ist, die mit der flüssigkeitsaufnehmenden Schicht (16) verbunden ist.

10. Schicht-Verbundstoff nach Anspruch 9, **dadurch gekennzeichnet, daß** die Abdeckschicht (18) mit der flüssigkeitsaufnehmenden Schicht (16) verklebt ist, wobei der Klebstoff an einer Mehrzahl von über der Fläche des Schicht-Verbundstoffs (10) verteilten, voneinander beabstandeten Klebepositionen (36) angeordnet ist.

## Claims

1. Planar, pliable laminated composite material, comprising a fluid-impermeable layer (14) and a fluid-absorbing layer (16) joined to the latter, **characterized in that** a base layer (12), which is a flexible mesh layer (34), carries the fluid-impermeable layer (14).

2. Laminated composite material according to claim 1, **characterized in that** the fluid-impermeable layer is a film (14).

3. Laminated composite material according to either of claims 1 or 2, **characterized in that** the fluid-absorbing layer (16) comprises a web (22), preferably of cotton fibres (24).

4. Laminated composite material according to claim 3, **characterized in that** the fluid-absorbing layer (16) comprises a fleece.

5. Laminated composite material according to either of claims 1 or 2, **characterized in that** the fluid-absorbing layer (16) comprises a nonwoven fabric layer.

6. Laminated composite material according to any one of the preceding claims, **characterized in that** the fluid-absorbing layer (16) comprises microcapsules (28) which are filled with an active substance (32) and comprise an enclosure (30) which can be degraded or destroyed by pressure, temperature or moisture.

7. Laminated composite material according to claim 6 in combination with claim 3, **characterized in that** the microcapsules (28) adhere to the fibres (24) of the web (22), preferably via a bonding agent (26).

8. Laminated composite material according to claims 1 to 7, **characterized in that** the flexible mesh layer (34) is of a material having a high friction, particularly of rubber.

9. Laminated composite material according to any one of the preceding claims, **characterized in that**, on the side facing away from the fluid-impermeable layer (14), there is provided for the fluid-absorbing layer (16) a fluid-permeable, preferably water-repellent cover layer (18) which is joined to the fluid-absorbing layer (16).

10. Laminated composite material according to claim 9, **characterized in that** the cover layer (18) is bonded to the fluid-absorbing layer (16), the adhesive being disposed at a plurality of bonding positions (36), separated from one another, distributed over the surface of the laminated composite material (10).

## Revendications

1. Matériau composite stratifié plan et flexible doté d'une couche imperméable aux liquides (14) et d'une couche absorbant les liquides (16) qui adhère à la précédente, **caractérisé en ce qu'**une couche de support (12) qui est une couche réticulée flexible (34) supporte la couche imperméable aux liquides (14).

2. Matériau composite stratifié selon la revendication 1, **caractérisé en ce que** la couche imperméable aux liquides est un film (14).

3. Matériau composite stratifié selon la revendication 1 ou 2, **caractérisé en ce que** la couche absorbant les liquides (16) présente un voile (22), de préférence en fibres de coton (24),

4. Matériau composite stratifié selon la revendication 3, **caractérisé en ce que** la couche absorbant les liquides (16) est constituée de molleton.

5. Matériau composite stratifié selon la revendication 1 ou 2, **caractérisé en ce que** la couche absorbant les liquides (16) est formée par une couche de non-tissé.

6. Matériau composite stratifié selon l'une des revendications précédentes, **caractérisé en ce que** la couche absorbant les liquides (16) présente des microcapsules (28) qui sont remplies d'une substance active (32) et présentent une enveloppe (30) pouvant être dégradée ou détruite par la pression, la température ou l'humidité.

7. Matériau composite stratifié selon la revendication 6 en relation avec la revendication 3, **caractérisé en ce que** les microcapsules (28) adhèrent aux fibres (24) du voile (22), de préférence par l'intermédiaire d'un liant (26).

8. Matériau composite stratifié selon l'une des revendications 1 à 7, **caractérisé en ce que** la couche réticulée flexible (34) est en une matière à fort frottement, notamment en caoutchouc.

9. Matériau composite stratifié selon l'une des revendications précédentes, **caractérisé en ce que**, sur la face opposée à la couche imperméable aux liquides (14), est prévue une couche de recouvrement perméable aux liquides, de préférence hydrophobe (18) pour la couche absorbant les liquides (16), qui est reliée à la couche absorbant les liquides (16).

10. Matériau composite stratifié selon la revendication 9, **caractérisé en ce que** la couche de recouvrement (18) est collée sur la couche absorbant les liquides (16), l'adhésif étant placé en de nombreux points d'adhésion (36) espacés les uns des autres et répartis sur la surface du matériau composite stratifié (10).
